# EUROPEAN PATENT APPLICATION

(11) **EP 4 541 408 A1**
(43) Date of publication of application: **23.04.2025**
(21) Application number: 23204814.0
(22) Date of filing: 20.10.2023
(51) Int. Cl.: A61M 25/10

(54) **CATHETER COMPRISING A SEGMENTED EXPANDABLE BODY AND AN EXPANDABLE COVER**

(71) Applicant: BIOTRONIK AG, 8180 Bülach (CH)
(72) Inventor: Göpfert, André, 18292 Alt Sammit (DE); Röthlisberger, André, 8820 Wädenswil (CH)
(74) Representative: Biotronik Corporate Services SE

(57) **Abstract**

The present invention relates to a catheter 1 comprising an expandable body 2 and a cover 3 for at least partially covering the expandable body 2, wherein the expandable body is attached to at least one catheter shaft 4 and wherein the expandable body 2 comprises at least two radially expandable segments 21, 22 and wherein the cover 3 is at least partially connected to the expandable body 2 and/or the at least one catheter shaft 4.

## Description

The invention refers to a catheter comprising a segmented expandable body and a cover for at least partially covering the expandable body. The invention further describes the use of the catheter in closing blood vessel perforations or artery ruptures, e.g. by using the cover as a temporary implant.

When using a standard PTA expandable body catheter for sealing purposes, the blood flow will be blocked or limited during covering of the dissection or perforation. Therefore, repeated and prolonged inflation and deflation of the expandable body is necessary to close the perforation. This is a time-consuming, labor- as well as resource-intensive procedure taking about 1 to 2 hours.

Using permanent implants may lead to irritation/inflammation of the vessel wall and/or stenosis. Vessels of coronary dimensions treated with covered stents are prone to long term severe clinical complications like myocardial infarction (MI), especially in case of synthetical covers.

Therefore, there is a need to further improve existing solutions for closing a vessel rupture/damage and to provide a catheter which can be used in time critical cases.

The aforementioned problems can be overcome by a catheter of claim 1. Particular features of the catheter are described in the dependent claims.

A catheter comprising an expandable body and a cover for at least partially covering the expandable body is described. The expandable body comprises at least two radially expandable segments, for example three, four, five or six radially expandable segments. The expandable body can be therefore denoted as segmented expandable body.

The (segmented) expandable body is inflatable and deflatable.

The segmented expandable body still allows for blood flow between the at least two radially expandable segments when the expandable body is inflated and has a low profile when being deflated.

The at least two radially expanding segments may be arranged (in regular or irregular spacing) around a circumference of a longitudinal catheter axis.

The expandable body may have a main body comprising the at least two radially expandable segments, for example three, four, five or six radially expandable segments, and the expandable body may have a distal cone that is situated at a distal expandable body end and/or a proximal cone that is situated at a proximal expandable body end, the distal cone and the proximal cone are adjacent to the main body of the expandable body.

Preferably, the expandable body is a balloon, preferably a percutaneous transluminal angioplasty (PTA) balloon.

The expandable body may be a thermoformed expandable body.

The inflation behavior and compliance of the expandable body can be adapted to the patient's needs. A high flexible expandable body for covering several inflation diameters might be preferred.

The expandable body is attached to at least one catheter shaft. The at least one catheter shaft may be an inner shaft extending through the expandable body and/or an outer shaft (having a larger diameter than a diameter of the inner shaft).

The at least two radially expandable segments, for example six radially expandable segments, may be distributed (equally) in a circumferential direction of the (inner) shaft.

The cover is (at least) partially connected/mounted to the expandable body and/or the at least one catheter shaft, for example via crimping, (laser-)welding, gluing, melting or molding.

The cover may be permanently connected to the expandable body and/or the at least one catheter shaft.

Alternatively, the cover may be releasably and/or reconnectably connected to the expandable body and/or the at least one catheter shaft (i.e. the cover is connectable to the expandable body and/or the at least one catheter shaft).

When the expandable body and the cover and/or the cover and the at least one catheter shaft (e.g. inner and/or outer shaft) are connected or connectable to each other a pull back of the cover is possible.

A non-permanent connection of the cover to the expandable body and/or the at least one catheter shaft enables temporary deployment and a later pull back of the cover or the usage of a shape memory spiral or ring above or within the cover.

The cover can be adapted to any need of the vessel and diameter. For example, the cover may comprise a drug. The cover may be a flexible tube or sheet that can be inserted into an artery.

Thus, a reusable and resheathable catheter for temporarily delivering and deploying a cover to close an artery rupture without limiting the blood flow is provided.

The cover may comprise or consist of a woven or non-woven fabric. The cover may be manufactured by established processes like dip coating, spray coating, spin coating, electrostatic coating, roller coating or by other suitable techniques like casting, extrusion or electrospinning. The cover shall have a level of elasticity adapted or customized to prevent it from rupturing or otherwise getting damaged upon expansion.

The cover may be a polymeric cover comprising or consisting of one or more synthetic and/or natural (co-)polymers, preferably thermoplastic (co-)polymers including, but not limited to, polyamide (PA), polyethylene (PE), polyethylene terephthalate (PET) polylactic acid (PLA), polyhdroxyalkanoates (PHA), polycaprolactones (PCL), polyphosphazenes, polyurethanes (PU), polytetrafluoroethylene (PTFE), expanded PTFE (ePTFE), natural rubber, chitosan, (nano-)cellulose, collagen, alginate, hyaluronic acid, gelatine or silk protein and/or their derivatives. The cover may comprise or consist of a biological tissue, such as mammalian pericardium, e.g. porcine, bovine or equine pericardium, preferably extracted from animals and suitably conditioned by downstream tissue treatment processes.

The cover may be non-permeable or semi-permeable to fluids, preferably body fluids such as blood. The cover may be made of a material which is resistant to an inner blood pressure of a patient (to which it is delivered).

The two radially expanding segments may be spaced apart from each other by a non-radially expanding or a less radially expanding segment. Less radially expanding segment means a segment which expands less in a radial direction than each of the radially expanding segments. Therefore, the blood can flow along or between non-radially expanding and/or the less radially expanding segments.

At least a part of the expandable segments may be connected to the cover.

At least a part of the expandable segments and/or at least a part of the less radially expanding segment(s) may not be connected to the inner shaft.

At least a part of the non-radially expanding segment(s) may be connected to the inner shaft.

The radially expandable segments and optionally the less expandable segments expand towards the vessel wall when the expandable body is inflated.

The segment(s) of the expandable body may be thermoformed segments. That means that the segments and/or the expandable body were formed by thermoforming in order to achieve a predefined shape of segments and/or the expandable body.

The catheter may further comprise at least one wire (on an outer surface of the expandable body) extending along a longitudinal axis of the expandable body and between two of the at least two radially expandable segments.

A sealing of the vessel wall is generally needed up to 48 hours in maximum. A permanent implant therefore is not needed. Therefore, any long-term effects such as vessel irritation/inflammation and/or stenosis may be avoided.

The use of the cover as a temporary implant for less than 48 hours implantation time, preferably less than 2 hours implantation time, is described.

Thus, the catheter may be used for closing blood vessel dissections, perforations or artery ruptures (e.g. by using the cover as a temporary implant).

Alternatively to the expandable body a stent with a releasably and/or reconnectably cover can be used as well in combination with the features described above.

Briefly, the cover will be delivered next to the vessel wall, then the expandable body is inflated and the rupture/damage the vessel wall is sealed.

The catheter should deploy the cover for closing a dissection or puncture of the vessel wall. The cover only needs to stay until the body cells close the perforation, puncture, rupture or dissection.

After sealing the vessel the cover can be retracted by deflating the expandable body and the catheter including the cover and the expandable body can be pulled out of the patient.

Due to the special shaped expandable body the blood flow will not be limited, this is for example essential in coronary vessels.

However, there is no limitation by location for placing the cover in the human body.

Thus, a method for temporary implantation of a cover is described comprising the following steps in the consecutive order:
- delivering the cover with the catheter as described above to the patient's site to be treated;
- inflating the expandable body;
- (optionally releasing the cover from the expandable body to obtain a released cover);
- deflating the expandable body; and
- retracting the catheter from the patient's body with (or without) the cover.

In case the cover was released from the expandable body and left in the patient, the method may further comprise the following steps:
- maneuvering the expandable body with the catheter to the released cover;
- recoupling the released cover to the expandable body by inflating the expandable body;
- deflating the expandable body; and
- retracting the catheter from the patient's body together with the cover.

The recoupling may be realized for example by providing the expandable body with a sticky outer surface to adhere the cover (or vice versa the cover with a sticky inner surface) or by providing the expandable body and/or the cover with fastening means (e.g. hooks/loops on the outer surface of the expandable body and/ or the inner surface of the cover).

The cover as temporary implant will be taken out when the perforation/rupture of the blood vessel is closed, for example after about 2 hours.

The following figures are provided to support the understanding of the present invention:
- Fig. 1: shows an embodiment of a catheter comprising a segmented expandable body,
- Fig. 2A: shows a cross-sectional view of a segmented expandable body segmented by wires,
- Fig. 2B: shows a side view of the segmented expandable body segmented by wires of Fig. 2A,
- Fig. 3A: shows a cross-sectional view of a segmented expandable body with thermoformed segments,

- Fig. 3B: shows a side view of the segmented expandable body with thermoformed segments of Fig. 3A,
- Fig. 4A: shows a cross-sectional view of a segmented expandable body with segments attached to the inner shaft,
- Fig. 4B: shows a side view of the segmented expandable body with segments attached to the inner shaft of Fig. 4A.

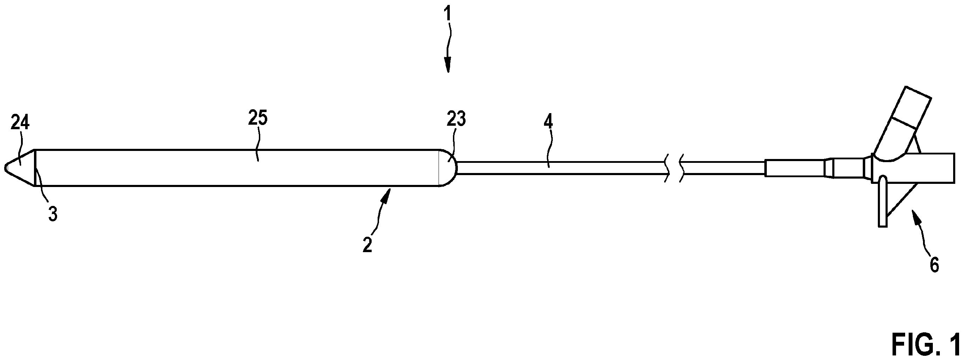

Fig. 1 shows an example of a catheter for delivering and deploying a cover to close an artery without limiting the blood flow.

The catheter 1 comprises a segmented expandable body 2, a cover 3 for at least partially covering the segmented expandable body, at least one catheter shaft 4 and a handle 6. The expandable body 2 is attached to at least one catheter shaft 4.

The segmented expandable body 2 comprises at least two radially expandable segments. The expandable body has a main body 25 comprising the at least two radially expanding segments and the expandable body has two cones 23, 24, each being adjacent to the main body 25. Thus, the expandable body has one distal cone 24 at its distal expandable body end and one proximal cone 23 at its proximal expandable body end. The distal cone 24 may have the same shape or a different shape than the proximal cone 23.

The cover 3 is at least partially connected to the expandable body 2 and/or the at least one catheter shaft 4. In detail, at least a part of the expandable segments is connected to the cover 3.

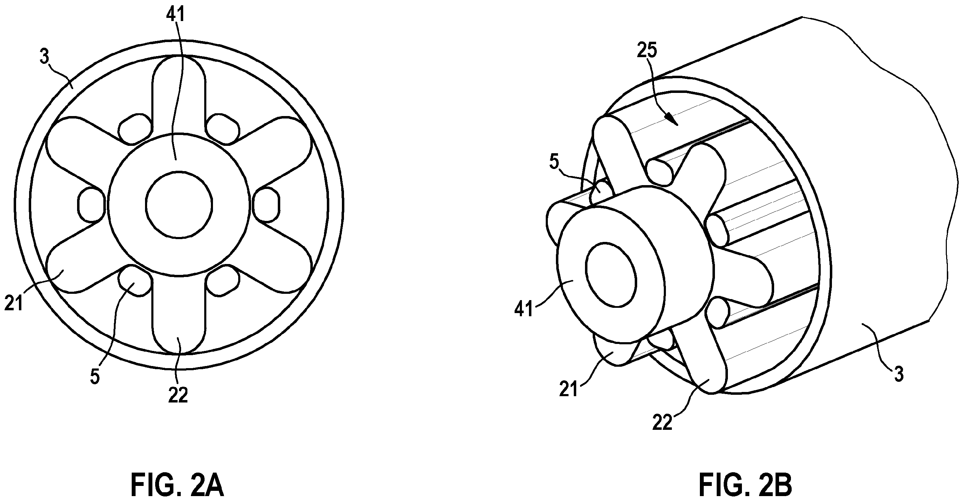

Fig. 2A and 2B show a segmented expandable body 2 for which the expansion of the expandable body 2 is restricted by wires 5 (mounted on the expandable body 2).

The expandable body 2 has six radially expanding segments 21, 22 and six wires 5 (arranged on an outer surface of the expandable body 2). Two radially expanding segments 21, 22 are spaced apart from each other by one wire 5.

The expandable body has a main body 25 comprising the radially expanding segments 21, 22. Alternatively, the expandable body may have two cones, each being adjacent to the main body. Thus, the expandable body may have one distal cone at its distal expandable body end and one proximal cone at its proximal expandable body end.

The expandable body 2 is attached at least to an inner shaft 41. The expandable body 2 might be additionally attached to an outer shaft (not shown).

A cover 3 is at least partially connected to the expandable body 2. In detail, at least a part of the expandable segments 21, 22 is connected to the cover 3.

The expandable body of Fig. 2A and 2B may be a part of the catheter described in Fig. 1.

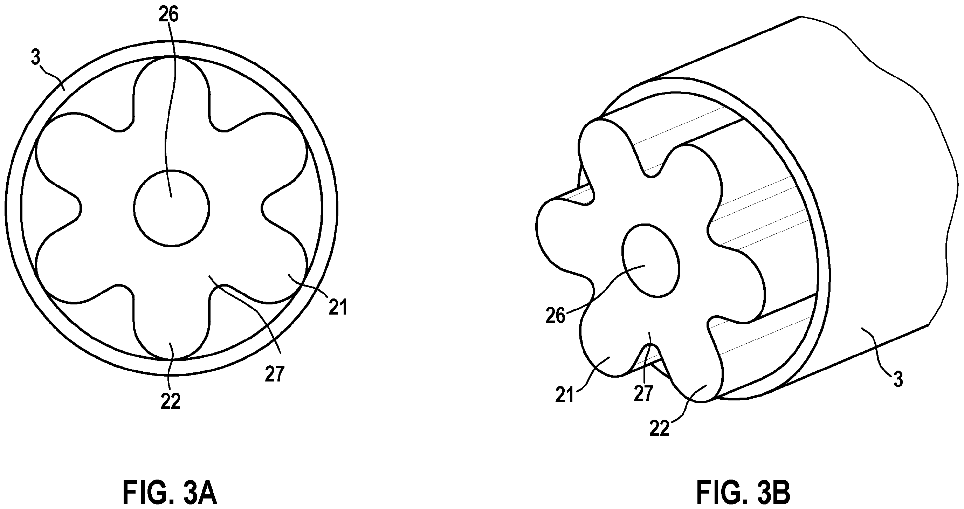

Fig. 3A and Fig. 3B show a segmented expandable body 2 for which the expansion of the expandable body 2 is restricted by its pre-defined form. The expandable body 2 is e.g. a thermoformed expandable body.

The segmented expandable body 2 has six radially expandable segments 21, 22 and six less radially expanding segments 27. Two radially expanding segments 21, 22 are spaced apart from each other by one less radially expanding segment 27.

The expandable body is attached to at least one catheter shaft, preferably an outer shaft (not shown).

A part of the expandable segments 21, 22 and a part of the less radially expanding segments 27 are not connected to an inner shaft. For, example because there might be no inner shaft. The expandable body 2 itself may form an inner body lumen 26.

The cover 3 is partially connected to the expandable body 2. At least a part of the expandable segments 21, 22 is connected to the cover 3.

The expandable body of Fig. 3A and 3B may be a part of the catheter described in Fig. 1.

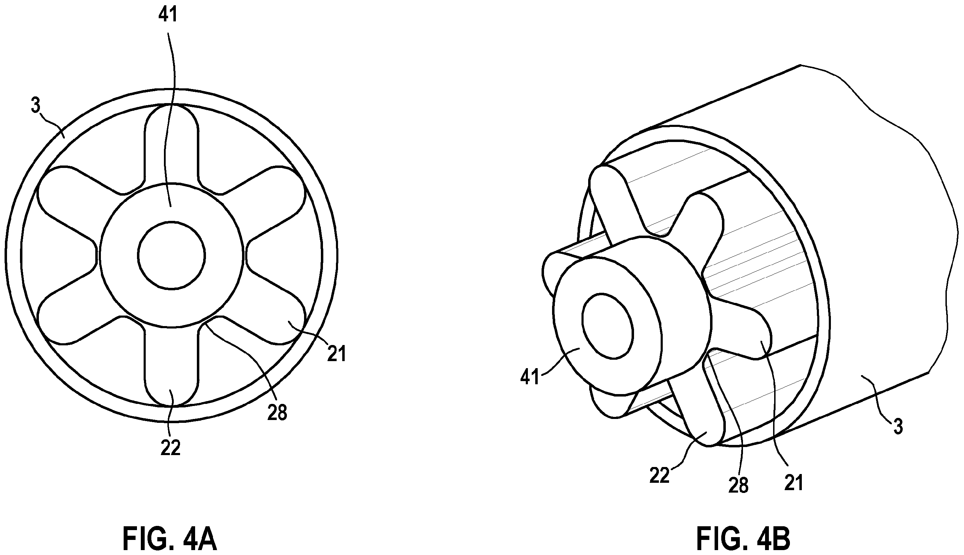

Fig. 4A and Fig. 4B show a segmented expandable body 2 for which the expansion of the expandable body 2 is restricted by mounting parts of the expandable body 2 to an inner shaft 41 protruding through the expandable body 2.

The expandable body 2 has six radially expandable segments 21, 22 and six non-radially expanding segments 28. Two radially expanding segments 21, 22 are spaced apart from each other by one non-radially expanding segment 28.

The expandable body has a main body comprising the radially expanding segments 21, 22 and the non-radially expanding segments 28. Alternatively, the expandable body may have two cones, each being adjacent to the main body. Thus, the expandable body may have one distal cone at its distal expandable body end and one proximal cone at its proximal expandable body end.

At least a part of the non-radially expanding segments 28 is connected to an inner shaft 41.

At least a part of the expandable segments 21, 22 of the expandable body is connected to a cover 3.

The expandable body of Fig. 4A and 4B may be a part of the catheter described in Fig. 1.

### List of reference signs

- 1: catheter
- 2: expandable body
- 3: cover
- 21, 22: radially expandable segments
- 23: proximal cone
- 24: distal cone
- 25: main body
- 26: inner body lumen
- 27: less radially expanding segment
- 28: non-radially expanding segment
- 4: catheter shaft
- 41: inner shaft
- 5: wire
- 6: handle

## Claims

1. A catheter (1) comprising an expandable body (2) and a cover (3) for at least partially covering the expandable body (2), wherein the expandable body is attached to at least one catheter shaft (4 )and wherein the expandable body (2) comprises at least two radially expandable segments (21, 22) and wherein the cover (3) is at least partially connected to the expandable body (2) and/or the at least one catheter shaft (4).

2. The catheter (1) according to claim 1, wherein the at least two radially expandable segments (21, 22) are arranged around a circumference of a longitudinal catheter axis.

3. The catheter (1) according to claim 1 or 2, wherein two radially expanding segments (21, 22) are spaced apart from each other by a non-radially expanding segment (28) or a less radially expanding segment (27).

4. The catheter (1) according to any one of the preceding claims, wherein the at least one catheter shaft (4) is an inner shaft (41) extending through the expandable body (2).

5. The catheter (1) according to any one of the preceding claims, wherein at least a part of the expandable segments (21, 22) is connected to the cover (3).

6. The catheter (1) according to claim 3, wherein at least a part of the expandable segments (21, 22) and/or at least a part of the less radially expanding segments (27) is/are not connected to the inner shaft (41).

7. The catheter (1) according to claim 3, wherein at least a part of the non-radially expanding segments (28) is connected to the inner shaft (41).

8. The catheter (1) according to any one of the preceding claims, wherein the cover (3) is permanently or releasably and/or reconnectably connected to the expandable body (2) and/or the catheter shaft (4).

9. The catheter (1) according to any one of the preceding claims, wherein the cover (3) is at least partially connected to the expandable body (2) and/or the catheter shaft (4) by crimping, laser welding, welding, gluing, melting or molding.

10. The catheter (1) according to any one of the preceding claims, wherein the expandable body (2) is a thermoformed expandable body.

11. The catheter (1) according to any one of the preceding claims, wherein the cover (3) is a polymeric cover or a biological tissue cover.

12. The catheter (1) according to any one of the preceding claims, wherein the cover (3) is non-permeable or semi-permeable for body fluids, preferably for blood.

13. The catheter (1) according to any one of the preceding claims, wherein the catheter (1) further comprises at least one wire (5) on an outer surface of the expandable body (2), and the at least one wire (5) extends along a longitudinal expandable body axis and between two of the at least two radially expandable segments (21, 22).

14. The catheter (1) according to any one of the preceding claims, wherein the expandable body (2) is a balloon, preferably a percutaneous transluminal angioplasty balloon.

15. The catheter (1) according to any one of the preceding claims for use in closing blood vessel perforations or artery ruptures.
